# EUROPÄISCHE PATENTSCHRIFT

(11) **EP 0 647 413 B1**
(45) Veröffentlichungstag und Bekanntmachung des Hinweises auf die Patenterteilung: **16.07.1997**
(21) Anmeldenummer: 94115497.3
(22) Anmeldetag: 30.09.1994
(51) Int. Cl.: A41C 3/14, A41F 1/00, A61F 2/52

(54) **Verschluss für Eingriffe in Bekleidungsstücke**
Closure means for garment inserts
Moyens de fermeture pour empiècements sur vêtements

(30) Priorität: 05.10.1993 DE 9315100 U
(43) Veröffentlichungstag der Anmeldung: 12.04.1995
(73) Patentinhaber: Dr. HELBIG GmbH & Co. ORTHOPÄDISCHE PRODUKTE KG, 83098 Brannenburg (DE)
(72) Erfinder: Weber-Unger, Georg, A-6330 Kufstein (AT)
(74) Vertreter: Haug, Dietmar, Dipl.-Ing.

(56) Entgegenhaltungen:
- DE-U- 8 701 925
- DE-U- 9 113 346
- US-A- 3 576 037

## Beschreibung

Die Erfindung betrifft einen Verschluß für Eingriffe in Bekleidungsstücken, mit einem an dem Bekleidungsstück befestigbaren, biegeweichen, dünnen, flachen Wandteil, das aus mehreren Wandstücken besteht, von denen jedes einen Überlappungsteil hat, der einen Überlappungsteil des benachbarten Wandstücks überlappt, wobei jeder Überlappungsteil eine Kante hat, von der ein Abschnitt am Überlappungsteil des benachbarten Wandstückes befestigt ist, und von der ein weiterer Abschnitt an dem Überlappungsteil des benachbarten Wandstückes lose anlegbar ist, um den Eingriff zu schließen, und von dem Überlappungsteil des benachbarten Wandstückes wegbewegbar ist, um den Eingriff zu öffnen.

Ein solcher Verschluß wird zum Beispiel bei einem Prothesenbüstenhalter verwendet, um eine in ein Körbchen des Büstenhalters eingelegte Brustprothese darin zu halten und eine hautfreundliche Trennung zwischen der Rückseite der Prothese und dem Körper der Trägerin zu schaffen. Bei einem solchen Verschluß ist das Wandteil am körperseitigen Rand des die Prothese aufnehmenden Körbchens des Büstenhalters angenäht. Bei diesem bekannten Verschluß besteht das Wandteil aus zwei Wandstücken, von denen jedes einen Überlappungsteil hat, der sich mit dem Überlappungsteil des anderen Wandstückes überlappt, wobei die lose an dem jeweiligen Überlappungsteil anliegenden Kantenabschnitte geradlinig und parallel zueinander verlaufen. Zum Einlegen und Herausnehmen der Prothese werden die beiden lose anliegenden Kantenabschnitte mit der Hand auseinandergedrückt, so daß sich eine schlitzförmige Öffnung ergibt, durch die die Prothese in das Körbchen des Prothesenbüstenhalters einlegbar bzw. aus ihm herausnehmbar ist. Da die Prothesenformen und -größen erhebliche Unterschiede aufweisen, ist das Hindurchführen der Prothesen durch die schlitzförmige Öffnung des Verschlusses bei manchen Prothesenformen oder -größen einfacher als bei anderen. So sind zum Beispiel kleine Prothesen mit einem kreisförmigen Grundriß einfacher durch die schlitzförmige Öffnung eines Verschlusses hindurchzuführen als solche, die seitliche Flügel haben und besonders groß sind.

Eine Prothesentasche dessen körperseitiges Wandteil aus zwei sich einander teilweise überlappenden Wandstücken besteht, ist z.B. aus der Druckschrift DE-U-91 13 346 bekannt.

Die Aufgabe der Erfindung besteht darin, einen gattungsgemäßen Verschluß so auszubilden, daß die Überlappungsteile in beliebige Richtungen möglichst gleichweit auseinanderbewegbar sind, um die sich ergebende Öffnung in Form und Größe variabler zu gestalten, wobei gewährleistet ist, daß die Überlappungsteile im geschlossenen Zustand des Eingriffs sicher aneinanderliegen.

Die Aufgabe der Erfindung wird gemäß dem Anspruch 1 dadurch gelöst, daß das Wandteil aus wenigstens drei Wandstücken besteht, von denen jedes zwei Überlappungsteile hat, von denen der eine über einem Überlappungsteil des einen benachbarten Wandstückes liegt, und von denen der andere unter einem Überlappungsteil des anderen benachbarten Wandstückes liegt, wobei die an den jeweiligen Überlappungsteilen im geschlossenen Zustand des Eingriffs lose anliegenden Kantenabschnitte sich in einem im wesentlichen in der Flächenmitte des Wandteils liegenden Punkt schneiden.

Der erfindungsgemäße Verschluß hat den Vorteil, daß die durch Auseinanderbewegen der Überlappungsteile zu bildende Öffnung nahezu eine beliebige Form mit jeweils maximaler Größe haben kann. Dadurch ergeben sich weitere Anwendungsmöglichkeiten für den Verschluß, so zum Beispiel ist der Verschluß auch bei Hosen- und Jackentaschen verwendbar.

Vorzugsweise sind die lose anliegenden Kantenabschnitte eines jeden Wandstücks geradlinig, wodurch die Herstellung der Wandstücke einfach gehalten wird.

Des weiteren ist vorzugsweise jedes Wandstück zweilagig und längs der jeweiligen lose anliegenden Kantenabschnitte gefaltet, wodurch ein gutes Rückstellvermögen der Wandstücke und ein stabiler Schließzustand der Wandstücke erzielt werden.

Weitere bevorzugte Ausführungen der Erfindung sind in den Ansprüchen 2-7 dargelegt.

Ein Ausführungsbeispiel der Erfindung ist in den Zeichnungen dargestellt und wird im folgenden näher beschrieben. Es zeigt
- Figur 1: eine Draufsicht auf einen Verschluß für einen Prothesenbüstenhalter,
- Figur 2: einen Schnitt durch den in Figur 1 gezeigten Verschluß längs der Linie A-A und
- Figur 3: eine schematische Darstellung eines mit dem Verschluß versehenen Körbchens des Prothesenbüstenhalters in der Perspektive.

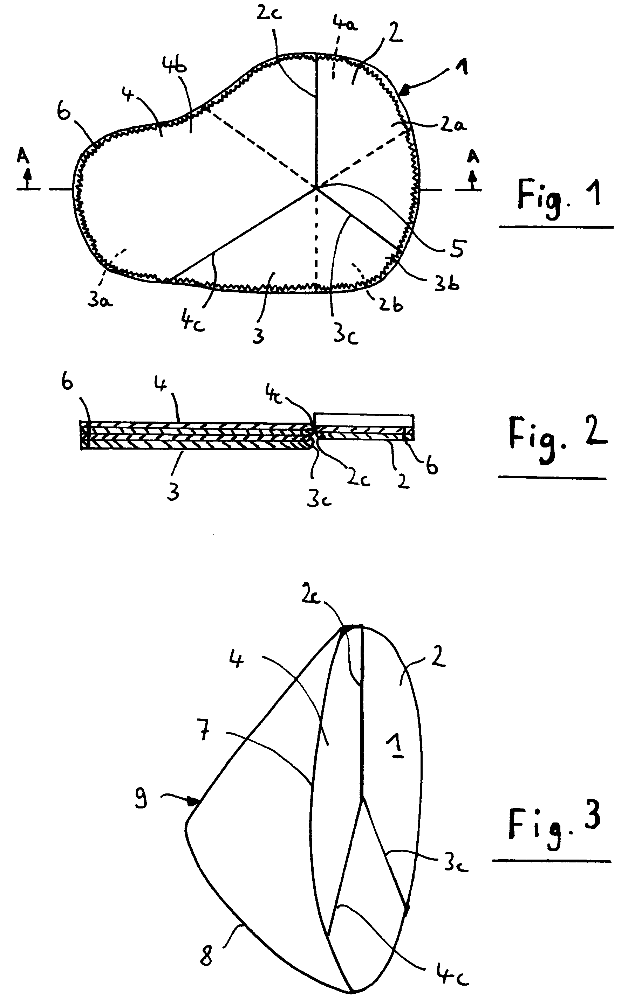

Der in den Zeichnungen dargestellte Verschluß besteht aus einem Wandteil 1, das aus drei Wandstücken 2, 3 und 4 aus Stoff, z.B. Baumwolle, zusammengesetzt ist. Jedes Wandstück hat zwei Überlappungsteile, von denen der eine über einem Überlappungsteil des einen benachbarten Wandstückes liegt, und von denen der andere unter einem Überlappungsteil des anderen benachbarten Wandstückes liegt. Im einzelnen hat das Wandstück 2 einen Überlappungsteil 2a, der einen Überlappungsteil 4a des Wandstückes 4 überlappt. Ferner hat das Wandstück 2 einen Überlappungsteil 2b, der unter einem Überlappungsteil 3b des Wandstückes 3 liegt. Das Wandstück 3 hat ferner einen Überlappungsteil 3a, der unter einem Überlappungsteil 4b des Wandstückes 4 liegt. Jeder Überlappungsteil hat einen geradlinigen Kantenabschnitt, der im geschlossenen Zustand des Eingriffs lose an dem benachbarten Wandstück anliegt. Die geradlinigen Kantenabschnitte schneiden sich in einem Punkt 5, der im wesentlichen in der Mitte der Fläche des Wandteils 1 liegt. Im einzelnen hat das Wandstück 2 einen geradlinigen Kantenabschnitt 2c, der zwischen seinem in der Figur 1 oberen Ende und dem Schnittpunkt 5 auf dem Wandstück 4 verläuft und zwischen dem Schnittpunkt 5 und seinem in Figur 1 unteren Ende unter dem Wandstück 3 verläuft. Das Wandstück 3 hat einen geradlinigen Kantenabschnitt 3c, der zwischen seinem in Figur 1 rechten unteren Ende und dem Schnittpunkt 5 auf dem Wandstück 2 verläuft und zwischen dem Schnittpunkt 5 und seinem in Figur 1 linken oberen Ende unter dem Wandstück 4 liegt. Das Wandstück 4 hat einen Kantenabschnitt 4c, der zwischen seinem in Figur 1 linken unteren Ende und dem Schnittpunkt 5 auf dem Wandstück 3 verläuft und zwischen dem Schnittpunkt 5 und seinem in Figur 1 oberen rechten Ende unter dem Wandstück 2 verläuft.

Durch eine am Rand des Wandteils 1 umlaufende Naht 6 sind die Wandstücke 2, 3 und 4 aneinander befestigt. Im einzelnen verbindet die Naht 6 das Wandstück 2 mit dem Wandstück 4 im Abschnitt zwischen dem in Figur 1 rechten oberen Ende des Kantenabschnitts 4c und dem oberen Ende des Kantenabschnitts 2c. Des weiteren verbindet die Naht 6 das Wandstück 4 mit dem Wandstück 3 im Abschnitt zwischen dem in Figur 1 linken oberen Ende des Kantenabschnitts 3c und dem linken unteren Ende des Kantenabschnitts 4c. Ferner verbindet die Naht 6 das Wandstück 3 mit dem Wandstück 2 im Abschnitt zwischen dem in Figur 1 unteren Ende des Kantenabschnitts 2c und dem rechten unteren Ende des Kantenabschnitts 3c.

Die Wandstücke 2, 3 und 4 sind jeweils zweilagig, wobei sie längs ihres jeweiligen geradlinigen Kantenabschnitts 2c, 3c bzw. 4c gefaltet sind. Die beiden Lagen eines jeden Wandstücks 2, 3 und 4 sind an ihren äußeren Enden durch die umlaufende Naht 6 miteinander verbunden.

Wie aus Figur 3 ersichtlich, ist das Wandteil 1 am körperseitigen Rand 7 eines Körbchens 8 eines Prothesenbüstenhalters 9 befestigt. Durch Auseinanderbewegen der geradlinigen Kantenabschnitte 2c, 3c und 4c wird eine Öffnung gebildet, durch die eine Brustprothese in das Körbchen 8 eingelegt bzw. aus ihm herausgenommen werden kann. Nach dem Loslassen der Wandstücke 2, 3 und 4 bewegen sie sich wieder weitgehend selbsttätig in die in der Zeichnung dargestellte Lage, in der sie den Eingriff in das Körbchen 8 verschließen, so daß die in dem Körbchen 8 sich befindende Prothese darin gehalten wird. Das Wandteil 1 bildet ferner eine hautfreundliche Trennwand zwischen der Prothese und dem Körper der Trägerin.

Durch den im geschlossenen Zustand des Eingriffs sternförmigen Verlauf der geradlinigen Kantenabschnitte 2c, 3c und 4c ist es möglich, eine Öffnung beliebiger Form und maximaler Größe zu schaffen, die fast bis zu der äußeren Begrenzungslinie des Wandteils 1 reicht. Infolgedessen ist das Einlegen und Herausnehmen der Prothese in das bzw. aus dem Körbchen bequem und einfach selbst bei solchen Prothesen, die relativ groß sind und im Grundriß von der Kreisform stark abweichen.

Der Verschluß kann auch bei einer Prothesentasche verwendet werden, die dazu dient, eine Brustprothese aufzunehmen, und mit dieser Brustprothese zusammen in das Körbchen eines Büstenhalters eingelegt zu werden. Bei Verwendung des Verschlusses einer solchen Prothesentasche wird das Wandteil am körperseitigen Rand der Tasche befestigt.

Der Verschluß könnte auch bei einem Badeanzug für Prothesenträgerinnen verwendet werden, indem das Wandteil am körperseitigen Rand des die Brustprothese aufnehmenden Körbchens des Badeanzugs befestigt wird.

## Patentansprüche

1. Verschluß für Eingriffe in Bekleidungsstücken, mit einem an dem Bekleidungsstück befestigbaren, biegeweichen, dünnen, flachen Wandteil (1), das aus mehreren Wandstücken (2, 3, 4) besteht, von denen jedes einen Überlappungsteil (2a, 2b; 3a, 3b; 4a, 4b) hat, der einen Überlappungsteil des benachbarten Wandstückes überlappt, wobei jeder Überlappungsteil eine Kante hat, von der ein Abschnitt (2c, 3c, 4c) am Überlappungsteil des benachbarten Wandstückes befestigt ist, und von der ein weiterer Abschnitt an dem Überlappungsteil des benachbarten Wandstückes lose anlegbar ist, um den Eingriff zu schließen, und von dem Überlappungsteil des benachbarten Wandstückes wegbewegbar ist, um den Eingriff zu öffnen, dadurch gekennzeichnet, daß das Wandteil (1) aus wenigstens drei Wandstücken (2, 3, 4) besteht, von denen jedes zwei Überlappungsteile (2a, 2b; 3a, 3b; 4a, 4b) hat, von denen der eine über einem Überlappungsteil des einen benachbarten Wandstückes liegt, und von denen der andere unter einem Überlappungsteil des anderen benachbarten Wandstückes liegt, wobei die an den jeweiligen Überlappungsteilen im geschlossenen Zustand des Eingriffs lose anliegenden Kantenabschnitte (2c, 3c, 4c) sich in einem im wesentlichen in der Flächenmitte des Wandteils (1) liegenden Punkt (5) schneiden.

2. Verschluß nach Anspruch 1, dadurch gekennzeichnet, daß die lose anliegenden Kantenabschnitte (2c, 3c, 4c) eines jeden Wandstücks (2, 3, 4) geradlinig sind.

3. Verschluß nach Anspruch 1 oder 2, dadurch gekennzeichnet, daß jedes Wandstück (2, 3, 4) zweilagig ist und längs der jeweiligen lose anliegenden Kantenabschnitte (2c, 3c, 4c) gefaltet ist.

4. Verschluß nach einem der vorhergehenden Ansprüche, dadurch gekennzeichnet, daß die Wandstücke (2, 3, 4) aus einem textilen Stoff bestehen.

5. Verschluß nach Anspruch 4, dadurch gekennzeichnet, daß das Wandteil (1) am körperseitigen Rand einer eine Brustprothese aufnehmenden Prothesentasche befestigt ist.

6. Verschluß nach Anspruch 4, dadurch gekennzeichnet, daß das Wandteil (1) am körperseitigen Rand (7) eines eine Brustprothese aufnehmenden Körbchens (8) eines Prothesenbüstenhalters (9) befestigt ist.

7. Verschluß nach Anspruch 4, dadurch gekennzeichnet, daß das Wandteil am körperseitigen Rand eines eine Brustprothese aufnehmenden Körbchens eines Badebekleidungsstücks befestigt ist.

## Claims

1. A closure for entry into articles of clothing, having a flexurally soft, thin, flat wall part (1) which is adapted to be fixed to the article of clothing and which consists of a plurality of wall pieces (2, 3, 4) each of which has an overlap part (2a, 2b; 3a, 3b; 4a, 4b), which overlaps an overlap part of the adjacent wall piece, each overlap part having an edge, a portion (2c, 3c, 4c) of which is fixed on the overlap part of the adjacent wall piece, and another portion of which is adapted to be loosely placed on the overlap part of the adjacent wall piece in order to close the entry, and is movable away from the overlap part of the adjacent wall piece in order to open the entry, characterised in that the wall part (1) consists of at least three wall pieces (2, 3, 4), each of which has two overlap parts (2a, 2b; 3a, 3b; 4a, 4b), one of which lies over an overlap part of an adjacent wall piece, and the other of which lies below an overlap part of the other adjacent wall piece, the edge portions (2c, 3c, 4c), which rest loosely against the associated overlap parts when the entry is closed, intersecting at a point (5) situated substantially in the middle of the surface of the wall part (1).

2. A closure according to claim 1, characterised in that the loosely contacting edge portions (2c, 3c, 4c) of each wall piece (2, 3, 4) are rectilinear.

3. A closure according to claim 1 or 2, characterised in that each wall piece (2, 3, 4) is in two layers and is folded along the respective loosely contacting edge portions (2c, 3c, 4c).

4. A closure according to any one of the preceding claims, characterised in that the wall pieces (2, 3, 4) consist of a textile material.

5. A closure according to claim 4, characterised in that the wall part (1) is fixed to a breast prosthesis pocket at the edge thereof facing the body.

6. A closure according to claim 4, characterised in that the wall part (1) is fixed to the edge (7), facing the body, of a cup (8) of a prosthesis brassiere (9), said cup receiving a breast prosthesis.

7. A closure according to claim 4, characterised in that the wall part is fixed to the edge, facing the body, of a bathing costume cup receiving a breast prosthesis.

## Revendications

1. Fermeture pour éléments de réception dans des pièces d'habillement, présentant une partie de paroi (1), mince, plate et souple, qui peut être fixée à la pièce de vêtement, qui est constituée de plusieurs pièces de paroi (2, 3, 4), qui présentent chacune une partie de superposition (2a, 2b; 3a, 3b; 4a, 4b) qui se superpose à une partie de superposition de la pièce de paroi voisine, dans laquelle chaque partie de superposition présente un bord dont une section (2c, 3c, 4c) est fixée à la partie de superposition de la pièce de paroi voisine, et dont une autre section peut être posée de manière flottante sur la partie de superposition de la pièce de paroi voisine pour fermer l'élément de réception et peut être écartée de la partie de superposition de la pièce de paroi voisine pour ouvrir l'élément de réception, caractérisée en ce que la partie de paroi (1) est constituée d'au moins trois pièces de paroi (2, 3, 4) dont chacune présente deux parties de superposition (2a, 2b; 3a, 3b; 4a, 4b), dont l'une est située au-dessus d'une partie de superposition de l'une des pièces de paroi voisine et dont l'autre est située en dessous d'une partie de superposition de l'autre pièce de paroi voisine, les sections de bord (2c, 3c, 4c) qui sont posées de manière flottante sur les parties de superposition concernées dans l'état fermé de l'ouverture de réception se coupant en un point (5) situé essentiellement au milieu de la surface de la partie de paroi (1).

2. Fermeture selon la revendication 1, caractérisée en ce que les parties de bord (2c, 3c, 4c) posées de manière flottante de chaque pièce de paroi (2, 3, 4) sont rectilignes.

3. Fermeture selon les revendications 1 ou 2, caractérisée en ce que chaque pièce de paroi (2, 3, 4) est constituée de deux couches et est repliée le long de la section de bord (2c, 3c, 4c) posée de manière flottante, correspondante.

4. Fermeture selon l'une des revendications précédentes, caractérisée en ce que les pièces de paroi (2, 3, 4) sont réalisées en un matériau textile.

5. Fermeture selon la revendication 4, caractérisée en ce que la partie de paroi (1) est fixée au bord tourné vers le corps d'une poche de prothèse reprenant une prothèse du sein.

6. Fermeture selon la revendication 4, caractérisée en ce que la partie de paroi (1) est fixée au bord (7), situé du côté du corps, d'un bonnet (8) d'un soutien-gorge de prothèse (9) reprenant une prothèse du sein.

7. Fermeture selon la revendication 4, caractérisée en ce que la partie de paroi est fixée au bord tourné vers le corps d'un bonnet d'un vêtement de bain reprenant une prothèse du sein.
